# EUROPÄISCHE PATENTSCHRIFT

(11) **EP 2 346 539 B1**
(45) Veröffentlichungstag und Bekanntmachung des Hinweises auf die Patenterteilung: **02.01.2013**
(21) Anmeldenummer: 09795896.1
(22) Anmeldetag: 30.10.2009
(51) Int. Cl.: A61L 27/14, A61L 27/16, A61C 13/087, A61K 6/083, C08F 265/06

(54) **POLYMERISIERBARES MEHRKOMPONENTEN-PROTHESENAUSGANGSMATERIAL, INSBESONDERE FÜR DENTALPROTHESEN**
POLYMERIZABLE MULTI-COMPONENT PROSTHESIS STARTING MATERIAL, PARTICULARLY FOR DENTAL PROSTHESES
MATÉRIAU DE DÉPART MULTICOMPOSANT POLYMÉRISABLE POUR PROTHÈSE, NOTAMMENT POUR PROTHÈSES DENTAIRES

(30) Priorität: 07.11.2008 DE 102008056293
(43) Veröffentlichungstag der Anmeldung: 27.07.2011
(73) Patentinhaber: Retec Kunststofftechnik GmbH, 61191 Rosbach v.d. Höhe (DE)
(72) Erfinder: REISS, Siegfried, 61191 Rosbach v.d. Höhe (DE)
(74) Vertreter: Metten, Karl-Heinz
(86) Internationale Anmeldenummer: PCT/DE2009/001521
(87) Internationale Veröffentlichungsnummer: WO 2010/051793

(56) Entgegenhaltungen:
- EP-A2- 1 923 037
- GB-A- 760 344
- US-A- 4 112 023
- US-B1- 6 843 654

## Beschreibung

Die vorliegende Erfindung betrifft ein polymerisierbares Mehrkomponenten-Prothesenausgangsmaterial, insbesondere ein polymerisierbares 2-Komponenten-Prothesenausgangsmaterial. Des Weiteren betrifft die Erfindung aus diesem Mehr- bzw. 2-Komponenten-Prothesenausgangsmaterial hergestellte Prothesenmaterialien und Prothesen, insbesondere Dentalprothesen.

An Prothesenmaterialien, insbesondere auch an Dentalprothesen, seien es Total- oder Teilprothesen, provisorische oder permanente Prothesen, wie Kronen, Brücken oder kieferorthopädische Apparate, sowie an Otoplastiken, so genannte Ohrpassstücke für die Hörgeräteindustrie, werden heutzutage sehr hohe Anforderungen in vielfältiger Hinsicht gestellt. Derartige Prothesen haben nicht nur gut bioverträglich zu sein, sondern sollen sich stets auch durch eine hohe Passgenauigkeit, vor allem während des dauerhaften Gebrauchs, geringe Beschädigungsneigung, geringen Volumenschrumpf und stets einwandfreie Oberflächen auszeichnen. Der Tragekomfort wird sowohl durch das äußere Erscheinungsbild als auch durch die sensorische Wahrnehmung der Prothese im Mundraum oder im Gehörgang geprägt.

Für Dentalprothesen steht dem Fachmann eine Vielzahl an geeigneten Materialien zur Verfügung. Gemäß dem Standard DIN EN ISO 1567 kann es sich hierbei um Heißpolymerisate auf der Basis von Pulver und Flüssigkeit, Heißpolymerisate auf der Basis eines plastischen Gemisches, Auto(kalt)polymerisate auf der Basis von Pulver und Flüssigkeit, Auto(kalt)polymerisate auf der Basis von Pulver und Flüssigkeiten für Gießkunststoffe, thermoplastisch formbare Rohlinge oder Granulate, lichthärtende Materialien und mikrowellenhärtende Materialien handeln. Die Grenze zwischen kalthärtenden und heißhärtenden Systemen liegt gemäß ISO1567:2000 bei 65°C.

Besonders bewährt haben sich im Dentalbereich 2-Komponenten-Materialien auf der Basis von Polymethylmethacrylaten (PMMA), und zwar sowohl als Heiß- als auch als Kaltpolymerisate.

Polymerisierbare 2-Komponenten-Materialien für den Dentalbereich auf Basis von PMMA sind aus der GB 760 344 bekannt.

Trotz erheblicher Bemühungen lassen auch die im Markt bekannten Materialien aufgrund der vorangehend geschilderten hohen Anforderungen noch stets Wünsche offen. Beispielsweise beobachtet man insbesondere bei Dentalprothesen aus Heißpolymerisaten einen nicht unerheblichen Volumenschrumpf sowie thermischen Schrumpf, was zu Passungenauigkeiten führt. Ein hoher Restmonomergehalt, welcher sowohl das Dimensionsverhalten von Prothesen negativ beeinflussen als auch sogar, z.B. wegen Schleimhautreizungen, zu Prothesenunverträglichkeiten führen kann, wird insbesondere bei der Kaltpolymerisation beobachtet. Um die von der DIN EN ISO 1567:2000 geforderten maximalen Restmonomergehalte von 2,2 % für Heiß- und für 4,5 % für Auto- bzw. Kaltpolymerisate einzuhalten, werden die Prothesen häufig für einen längeren Zeitraum in Wasser gelagert, was jedoch wieder zu Dimensionsveränderungen führen kann.

Von nicht geringer Bedeutung für die Güte einer Zahnprothese ist ebenfalls deren mechanisches Verhalten. Um bei Beanspruchungsspitzen nicht sogleich beschädigt zu werden, wird von heutigen Zahnprothesen eine hohe Bruch-, Biege- und Schlagfestigkeit gefordert.

Schließlich ist insbesondere bei Zahnprothesen auf eine besonders hohe Oberflächenqualität zu achten, denn ansonsten schafft man vielfältige Stellen für die Ablagerung von Mikroorganismen, was zur so genannten Plaque-Bildung führt. Auch ist es häufig sehr unvorteilhaft oder gar unmöglich, Oberflächenunebenheiten mittels Polierens zu bearbeiten, jedenfalls für solche Dentalprothesenareale, die bei gattungsgemäßem Gebrauch Ansaugflächen darstellen.

Es hat bislang im Stand der Technik nicht an Versuchen gefehlt, optimierte Zahnprothesen zu erhalten. Zahnprothesen mit verbesserter Schlag- bzw. Bruchfestigkeit auf der Basis von Polymethylmethacrylaten werden gemäß der EP 1 923 037 A2 dann erhalten, wenn man von einer flüssigen Monomerkomponente aus Methylmethacrylat ausgeht, welche zusätzlich ein (meth)acryliertes Butadien-Oligomer oder -Polymer oder ein (meth)acryliertes Acrylnitril-Butadien-Oligomer oder -Polymer enthält. Bei Reaktion der genannten Flüssigkomponente mit einer Pulverkomponente, enthaltend ein Polymerpulver oder ein Perlpolymerisat auf Methacrylatbasis, sollen Formteile zugänglich sein mit einer Transparenz größer 70 % bei einer Schichtdicke von 3 mm, die sich zudem durch eine hohe Schlagzähigkeit auszeichnen.

Zahnprothesen mit hoher Bruchfestigkeit sollen gemäß der EP 1 702 633 A2 im Wege eines autopolymerisierenden Mehrkomponenten-Prothesenausgangsmaterials erhalten werden, welches aus einer flüssigen Monomerkomponente und einer pulverförmigen füllstoffhaltigen Komponente besteht, die mindestens ein durch eine elastische Phase modifiziertes Perlpolymerisat enthält. Der nach dem Aushärten eines solchen Prothesenbasismaterials erhältliche Prothesenkunststoff verfügt gemäß der EP 1 702 633 A2 über eine Bruchzähigkeit von ≥ 2 MPa x m^{0.5} und eine Brucharbeit von ≥ 900 J/m². Geeignete elastische Phasen sollen dabei ausgewählt werden können aus der Gruppe bestehend aus Poly-(n-butylacrylat), Butadien/Styrol-Copolymer und Silikonkautschuk-(Pfropfcopolymerisaten).

Das Elastizitätsverhalten von Dentalformmassen aus Polymethacrylaten lässt sich gemäß der GB 1,584,530 durch Verwendung einer Polyurethanphase verbessern. Einen ähnlichen Ansatz findet man in der DE 27 19 149 OS offenbart.

Aus der WO 99/42078 A2 geht eine härtbare Dentalmasse hervor, die über Si-O-Si-Gruppen vernetzte Perlpolymerisate mit mittleren Teilchengrößen von 1 bis 10 µm verfügt. Derartige Dentalmassen sollen eine hohe Verschleißfestigkeit sowie eine glatte hochglänzende Oberfläche aufweisen.

Verbesserte Dentalformmassen sind gemäß der DE 10 2006 010 075 A1 zugänglich bei Verwendung einer flüssigen Monomerkomponente auf Methacrylat-Basis und einem in phlegmatisierter Form vorliegenden Initiator. Die Phlegmatisierung, d.h. die Herabsetzung der Reaktivität des Initiators, beispielsweise eines Peroxids, soll durch Einkapselung desselben in ein Perlpolymerisat möglich sein.

Dentalformkörper mit verringerter Sprödigkeit, verbessertem zähelastischen Verhalten und hoher Oberflächengüte lassen sich gemäß der EP 1 502 571 A1 bei Verwendung von Mischungen erhalten, welche ausgestattet sind mit einem monofunktionellen (Meth)acrylat, einem vernetzenden (Meth)acrylat, einem Splitterpolymer aus einem vernetzenden (Meth)acrylat und pyrogener Kieselsäure, einem teilweise vernetzten Perlpolymerisat, einem anorganisch verstärkten Perlpolymer, einem Initiator sowie Farbpigmenten. Als wesentlich für die verbesserten Dentalformkörper wird in der EP 1 502 571 A1 die Anwesenheit eines auf Methacrylat basierenden Perlpolymers hervorgehoben, in welches anorganisches Dentalglas als Füllstoff einpolymerisiert ist. Mit Hilfe anorganischer Füllmaterialien, insbesondere mit einer durchschnittlichen Partikelgröße kleiner 1 µm, versucht auch die US 4,308,190 zu verbesserten Dentalmaterialien auf der Basis von Methacrylaten zu gelangen. Ein ähnlicher Ansatz wird in der DE 24 03 211 US beschrieben, wonach anorganische Füllstoffe mit einer durchschnittlichen Teilchengröße kleiner 0,7 µm zu Dentalformkörpern mit verbesserten mechanischen und optischen Eigenschaften führen.

Die DE 100 65 501 A1 offenbart ein Verfahren zur Herstellung von Perlpolymerisaten mit einer mittleren Teilchengröße im Bereich von 1 bis 40 µm, bei dem man eine polymerisierbare Zusammensetzung, die mindestens 50 Gew.-% (Meth)acrylate aufweist, in wässriger Phase dispergiert und polymerisiert. Die Dispersion hat hierbei mit einer Aluminiumverbindung stabilisiert und mit einer Schergeschwindigkeit von mindestens 10³ s⁻¹ hergestellt zu werden. Auf diese Weise sollen Perlpolymerisate mit einer mittleren Teilchengröße im Bereich von 1 bis 40 µm erhalten werden, die bei Einarbeitung in Formmassen eine geringe Gelbfärbung zeigen. Auch soll man bei der Herstellung mit geringen Mengen an organischen Lösungsmitteln auskommen.

Aus der DE 940 493 B geht ein Verfahren zur Herstellung von z. B. Zahnprothesen aus polymerisierbaren organischen Verbindungen hervor. Hierbei werden fein verteilte polymere Verbindungen mit polymersierbaren flüssigen Monomeren oder teilweise polymerisierten Verbindungen zu einer plastischen, knetbaren Masse vermischt. Als fein verteilte polymere Verbindungen haben Mischungen von mindestens zwei verschiedenen fein verteilten Polymerisaten verwendet zu werden. Hierbei kann es sich beim ersten Bestandteil um ein Perlpolymerisat von Methacrylsäuremethylestern und beim zweiten Bestandteil um Mischpolymerisate von Methacrylsäuremethylestern mit Butadien oder dessen Homologen oder Derivaten bzw. Methacrylsäuremethylestern von Alkoholen mit mehr als vier Kohlenstoffatomen oder um feingepulvertes nachchloriertes Polyvinylchlorid handeln. Indem man die vorangehend beschriebenen Mischungen an Polymerisaten mit den ebenfalls vorangehend beschriebenen flüssigen Monomeren anquillt, erhält man gemäß der DE 940 493 B ein gut anteigbares Material, das erst nach längerer Zeit in dem knetbaren plastischen Zustand übergeht und diesen Zustand über einen längeren Zeitraum beibehält.

Die DE 24 08 640 A1 betrifft eine polymerisierbare Polymer-Monomer-Mischung mit einem polymerisierbaren Acrylmonomer als erstem Bestandteil sowie bis zu 30 Gew. % an einem gelösten, mit dem Monomer mischbaren und mit dem polymerisierten Monomer verträglichen Polymer als zweitem Bestandteil sowie einem teilchenförmigen, in dem Monomer dispergierten und mit dem Monomer verträglichen und von diesem benetzbaren Polymer als drittem Bestandteil. Mit diesen Ausgangsmaterialien soll ein wesentlich verringerter Schrumpf beim Aushärtprozess einhergehen.

Die aus dem Stand der Technik bekannten Dentalprothesen lassen noch stets Wünsche offen, beispielsweise hinsichtlich hoher Bruchfestigkeitswerte bei gleichzeitig geringem Restmonomeranteil. Demgemäß lag der vorliegender Erfindung die Aufgabe zugrunde, verbesserte Prothesenmaterialien, insbesondere Dentalprothesenmaterialien zur Verfügung zu stellen, welche nicht mit den Nachteilen des Stands der Technik behaftet sind und die insbesondere über eine hohe Verträglichkeit beim Nutzer und zugleich über besonders gute mechanische Eigenschaften verfügen.

Demgemäß wurde ein polymerisierbares Mehrkomponenten-Prothesenausgangsmaterial, insbesondere 2-Komponenten-Prothesenausgangsmaterial, gefunden, umfassend
a) mindestens eine flüssige Komponente A, enthaltend mindestens ein Alkylmethacrylat, insbesondere Methylmethacrylat,
b) mindestens eine Feststoffkomponente B, enthaltend
   i) mindestens ein erstes Homo- und/oder Copolymer eines Alkyl(meth)acrylats, insbesondere des Methylmethacrylats, in Pulverform und/oder in Form von Perlen mit einer ersten durchschnittlichen Partikelgröße (auch erstes (Co)polymerisat genannt) und
   ii) mindestens ein zweites Homo- und/oder Copolymer eines Alkyl(meth)acrylats, insbesondere des Methylmethacrylats, in Perlenform mit einer zweiten durchschnittlichen Partikelgröße (auch zweites (Co)polymerisat genannt), wobei die Perlen dieses zweiten (Co)polymerisats zumindest teilweise vernetzt vorliegen,
   wobei die erste durchschnittliche Partikelgröße größer als die zweite durchschnittliche Partikelgröße ist, wobei die erste durch schnittliche Partikelgröße zwischen 30 µm und 125 µm liegt und wobei die zweite durchschnittliche Partikelgröße im Bereich von 0,1 µm bis 15 µm liegt.

Als Mehr- bzw. 2-Komponenten-Prothesenausgangsmaterialal wird im Sinne der vorliegenden Erfindung eine solche Zusammensetzung verstanden, aus der Prothesenmaterialien bzw. Prothesen oder Bestandteile hiervon, beispielsweise Dentalprothesen oder Otoplastiken zugänglich sind. Geeignete Dentalprothesenmaterialien, die aus den erfindungsgemäßen Prothesenausgangsmaterialien zugänglich sind, umfassen insbesondere die Prothesenbasis, welche mit verbesserten Eigenschaften ausgestattet werden kann. Die Prothesenbasis umfasst bei Dentalprothesen solche Bereiche bzw. Bauteile, in die die Zahnprothesen eingearbeitet werden und die letztendlich für die Anbindung an Areale des Gaumens vorgesehen sind.

Die Feststoffkomponente B stellt regelmäßig ein Schüttgut dar, das vorzugsweise in pulverförmiger oder rieselfähiger Konsistenz vorliegt.

Die Teilchen- bzw. Partikelgröße der ersten und zweiten (Co)polymerisate bezieht sich auf den Partikeldurchmesser und kann beispielsweise durch dem Fachmann bekannte Verfahren wie das Laserextinktionsverfahren oder mit Hilfe von Rasterelektronenmikroskopaufnahmen ermittelt werden. Exemplarisch sei hierfür auf die WO 2006/117041 A1 verwiesen.

Das Gewichtsverhältnis der Feststoffkomponente B zu der Flüssigkomponente A liegt vorteilhafterweise im Bereich von 10:8 bis 10:2, vorzugsweise im Bereich von 10:6 bis 10:2 und insbesondere im Bereich von 10:5,5 bis 10:2,5. Es lassen sich beispielsweise ohne weiteres Gewichtsverhältnisse von 10:4 oder 10:3 einstellen. Indem man auf Mischungen zurückgreifen kann mit einem geringen Anteil an Flüssigkomponente, d.h. des zu polymerisierenden Monomers, resultiert mit den erfindungsgemäßen Prothesenausgangsmaterialien stets ein sehr geringer Restmonomergehalt in den hergestellten Prothesenmaterialien, und zwar sowohl mit Heiß- als auch mit Kaltpolymerisaten.

Während das erste Homo- und/oder Copolymer (Komponente i)) in einer besonders zweckmäßigen Ausgestaltung zu mindestens 80 Gew.- % in der Feststoffkomponente B vorhanden ist, liegt darin dass zweite Homo- und/oder Copolymer (Komponente ii)) in Mengen von maximal 20 Gew.-%, insbesondere nicht mehr als 15 Gew.-%, vor.

Die erste durchschnittliche Partikelgröße des ersten (Co)polymerisats liegt oberhalb von 30 µm, und die zweite durchschnittliche Partikelgröße des zweiten (Co)polymerisats liegt im Bereich von 0,1 µm bis 15 µm.

Die erste durchschnittliche Partikelgröße der Perlen des ersten Homo- und/oder Copolymers (erstes Copolymerisat) liegt im Bereich von 30 µm bis 125 µm, insbesondere im Bereich von 35 µm bis 80 µm. Die zweite durchschnittliche Partikelgröße der Perlen des zweiten (Co)polymerisats ist nicht größer als 15 µm, insbesondere kleiner 12 µm. Die ersten und insbesondere die zweiten durchschnittlichen Partikelgrößen sind bevorzugt eng verteilt.

Perlpolymerisate, insbesondere solche aus (Meth)acrylaten, sind dem Fachmann im allgemeinen bekannt. Perlpolymerisate auf der Basis von Polyalkyl(meth)acrylaten, insbesondere Polymethylmethacrylaten, vor allem solche mit kleinen durchschnittlichen Partikelgrößen, beispielsweise im Bereich von 1 µm bis 30 µm, lassen sich z. B. mittels Fällungspolymerisation erhalten. Im Wege der Suspensionspolymerisation sind üblicherweise Perlpolymerisate mit durchschnittlichen Teilchengrößen größer als 35 µm ohne weiteres zugänglich. Exemplarisch sei in diesem Zusammenhang auf die Offenbarung der EP 0 443 609 A2 verwiesen, auf welche hiermit ausdrücklich Bezug genommen wird. Gemäß der DE 100 65 501 A1 gelangt man mittels Suspensionspolymerisation zu Perlpolymerisaten mit durchschnittlichen Partikelgrößen im Bereich von 1 µm bis 40 µm, wenn die polymerisierbare Zusammensetzung mindestens 50 Gew.-% (Meth)acrylate aufweist und wenn in wässriger Phase dispergiert und polymerisiert wird. Die Suspension ist mit Hilfe von Aluminiumverbindungen zu stabilisieren. Vernetzte Perlpolymerisate mit einem mittleren Partikeldurchmesser von 10 bis 100 µm auf der Basis von Methacrylatpolymeren entnimmt man DE 28 50 916. Als geeignete multifunktionelle Vernetzermoleküle werden dort Ethylenglycoldimethacrylat und Divinylbenzol explizit genannt.

Für das in Perlenform vorliegende erste (Co)polymerisat, auch erstes Perlpolymerisat genannt, und/oder für das zweite (Co)polymerisat, auch zweites Perlpolymerisat genannt, wird als Hauptkomponente vorzugsweise auf Methylmethacrylat zurückgegriffen.

Daneben können auch weitere Comonomere einpolymerisiert sein. Exemplarisch seien als monofunktionelle Comonomere genannt Styrol, alpha-Methylstyrol, Vinyltoluol, substituierte Vinyltoluole wie Vinylbenzylchloride, Butadien, Isobutylen, 2-Chlorbutadien, 2-Methylbutadien, Vinylpyridin, Cyclopenten, sowie (Meth)acryl-säureester wie Methylmethacrylat, Butylmethacrylat, Butylacrylat und Hydroxyethylmethacrylat, ferner Acrylnitril, Vinylacetat und Vinylpropionat sowie Mischungen dieser Comonomere. Zu den bevorzugten Comonomeren gehören unter anderen Vinylalogenide wie Vinylchlorid, Vinylester, wie Vinylacetat, heterocyclische Vinylverbindungen wie 2-Vinylpyridin, Maleinsäure und Maleinsäurederivate, wie beispielsweise Maleinsäureanhydrid, Fumarsäure und Fumarsäurederivate wie Fumarsäureester, Acrylsäure, Methacrylsäure sowie Aryl(meth)acrylate wie Benzylmethacrylat oder Phenylmethacrylat.

In einer bevorzugten Ausgestaltung sind in die Perlen des ersten (Co)polymerisats und in die Perlen des zweiten (Co)polymerisats zumindest teilweise Vernetzer einpolymerisiert worden. Die ersten und zweiten Perlpolymerisate umfassen somit auch vernetzte und teilvernetzte Perlpolymerisate.

Für die Vernetzung greift man regelmäßig auf multifunktionelle Comonomere oder auch multifunktionelle Oligomere zurück. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, außerdem Glycerindi(meth)acrylat, 2,2-bis [(gamma-methacryloxy-beta-oxypropoxy)-phenylpropan], bis-GMA, Bisphenol-A-dimethacrylat, Neopentylglycol-di(meth)acrylat, 2,2-di-methacryloxypolyethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat und Urethandimethacrylat, sowie di- oder trivinylische Verbindungen wie Divinylbenzol hervorgehoben. Selbstverständlich können auch Mischungen der genannten Vernetzermoleküle zum Einsatz kommen. Besonders geeignet sind auch solche multifunktionellen Verbindungen, insbesondere di- und/oder trifunktionelle Verbindungen, die elastische Einheiten besitzen und demgemäß geeignet sind, die aus den Prothesenausgangsmaterialien erhaltenen Prothesenmaterialien mit flexiblen Eigenschaften auszustatten.

Selbstverständlich kann nicht nur in der Komponente ii), sondern ebenfalls in der Komponente i) ein Vernetzer, z. B. wie vorangehend für die Komponente ii) beschrieben, einpolymerisiert sein. Diese Vernetzermoleküle können in den Komponenten i) bzw. ii) vorzugsweise im Bereich von 0.1 Gew.- % bis 10 Gew.- %, insbesondere im Bereich von 0,5 Gew.- % bis 5 Gew.- %, vorliegen.

Gemäß einer weiteren bevorzugten Ausführungsform stellen die Komponenten i) und/oder ii) ein mit mindestens einer elastischen Phase modifiziertes Perlpolymerisat dar. Besonders bevorzugt umfasst die Komponente ii) ein mit mindestens einer elastischen Phase modifiziertes Perlpolymerisat. Die elastische Phase wird bevorzugt ausgewählt aus der Gruppe bestehend aus Poly-(n-butylacrylat), Butadien-Styrol-Copolymeren, Silikonkautschuk-Pfropfcopolymerisaten, Urethan(meth)acrylaten, acryliertem oder methacryliertem Butadien-Oligomer oder -Polymer, und acryliertem oder methacryliertem Acrylnitril-Butadien-Oligomer oder -Polymer oder Mischungen dieser Komponenten. Die mit derartigen elastischen Phasen ausgestatteten Perlpolymerisate ermöglichen es, Prothesenmaterialien mit flexiblen Eigenschaften zu erhalten.

Die elastische Phase kann in dem Perlpolymerisat in verschiedenen Formen vorliegen. In Frage kommen z.B. ein elastischer Kern in einer festen Schale, mehrere elastische Kerne in einer festen Matrix oder mehrere so genannte Kern/Schale-Teilchen in einer festen Matrix. Auch ist es möglich, dass die elastische und die feste Phase ein interpenetrierendes Netzwerk bilden.

Als geeignete Alkylmethacrylate für die flüssige Komponente A sei auf Methyl-, Ethyl-, n-Propyl, i-Propyl-, n-Butyl, t-Butyl, i-Butyl, Benzyl- und Furfurylmethacrylate oder deren Mischungen verwiesen. Methylmethacrylat ist besonders bevorzugt als Monomer für die Flüssigkomponente A.

In einer zweckmäßigen Ausgestaltung liegt in der flüssigen Komponente A neben mindestens einem der vorangehend genannten monofunktionellen Alkylmethacrylatmonomere mindestens ein Vernetzer vor. Grundsätzlich kann für die Flüssigkomponente A auf diejenigen Vernetzer zurückgegriffen werden, wie vorangehend für die Komponenten B)i) und B)ii) beschrieben. Exemplarisch sei auf die Dimethacrylate wie 1,4-Butandiol-dimethacrylat verwiesen. Solche Vernetzermoleküle können in der Flüssigkomponente A in Mengen im Bereich von 0,1 bis 20 Gew.- %, vorzugsweise im Bereich von 1 bis 10 Gew.- %, beispielsweise 5 Gew.- % zugegen sein.

In einer bevorzugten Ausgestaltung des erfindungsgemäßen Prothesenausgangsmaterials ist vorgesehen, dass das zweite (Co)polymerisat gebildet ist aus einer Comonomermischung, umfassend Methylmethacrylat, n-Butylacrylat und mindestens ein Vernetzercomonomer. Als Vernetzercomonomere kommen z.B. die vorangehend für die Komponenten A), B)i) und B)ii) spezifizierten Vernetzer sowie deren beliebige Mischungen ausdrücklich in Betracht. Bevorzugt wird auf Ethylenglykoldimethacrylat zurückgegriffen.

In einer weiteren Ausführungsform können in der Flüssigkomponente A neben z.B. Methylmethacrylat als bevorzugtem Hauptmonomer (> 50 Gew.-%) weitere Comonomere vorliegen.

Das für die Polymerisation erforderliche radikalische Initiatorsystem ist, je nach Reaktionsbedingungen bzw. Polymerisationssystem, in der flüssigen Komponente A und/oder der Feststoffkomponente B enthalten. Diesbezügliche Details sind dem Fachmann bekannt. Beispielsweise liegt bei Basismischungen für Kaltpolymerisate das Initiatorsystem meist in beiden Komponenten, der flüssigen Komponente und der Feststoffkomponente vor und wird demgemäß beim Mischen dieser Komponenten zusammengeführt. Bei den Basismaterialien für Heißpolymerisate liegt der Initiator meist in der Polymerkomponente, d.h. der Feststoffkomponente vor. Erst beim Mischen gelangt der Initiator dann in die flüssige Monomerkomponente. Demgemäß können Prothesenausgangsmaterialien vorgesehen sein, bei denen in der Feststoffkomponente B) i) und/oder der Feststoffkomponente B ii) ein Gehalt an reaktiver Initiatorkomponente, insbesondere in Form von Peroxiden, Perketalen, Perestem und/oder Azoverbindungen, vorliegt. Hierbei kann es sich z.B. auch um Restgehalte an bei der Herstellung der Feststoffkomponenten nicht abreagierter Initiatorkomponente handeln, z.B. um Peroxide wie Dibenzoylperoxid.

Als Initiatoren für die Polymerisationsreaktion von Kalt- wie auch Heißpolymerisat-Ausgangsmischungen kommen grundsätzlich solche in Betracht, mit denen sich radikalische Polymerisationsreaktionen starten lassen.

Bevorzugte Initiatoren sind Peroxide wie Dibenzoylperoxid, Dilauroylperoxid, t-Butanperbenzoat und Di-t-butyl-peroxid sowie Azoverbindungen wie Azobis(isobutyronitril)

(AIBN) oder Azobis(4-cyanvaleriansäure). Für die Heißhärtung kann des Weiteren auch auf Perketale und Benzpinakole zurückgegriffen werden.

Um die Initiierung der radikalischen Polymerisation durch Peroxide zu beschleunigen, können geeignete Aktivatoren, z.B. aromatische Amine, hinzugefügt werden. Exemplarisch seien als geeignete Amine N,N-Dimethyl-p-toluidin, N,N-Dihydroxyethyl-p-toluidin und p-Dibenzylaminobenzoesäurediethylester genannt. Hierbei fungieren die Amine regelmäßig als Co-Initiatoren und liegen üblicherweise in einer Menge von bis zu 0,5 Gew.- % vor.

Als radikalische Initiatorsysteme sind weiterhin Redoxsysteme geeignet, insbesondere Kombinationen aus Dibenzoylperoxid, Dilauroyl oder Campherchinon mit Aminen wie N, N-Dimethyl-p-toluidin, N-N-Dihydroxyethyl-p-toluidin und p-Dimethylaminobenzoesäurediethylester. Ferner können als Redoxsysteme auch solche zum Einsatz kommen, die neben einem Peroxid auch noch Ascorbinsäure oder deren Derivate, Barbitursäure oder ein Barbitursäurederivat oder eine Sulfinsäure als Reduktionsmittel enthalten. In einer zweckmäßigen Ausführungsform enthält ein solches Redoxsystem Barbitursäure oder Thiobarbitursäure oder ein Barbitursäure- oder Thiobarbitursäurederivat (beispielsweise 25 bis 80 Gew.- %), mindestens eine peroxidische Verbindung, z.B. eine Peroxodisulphat- und/oder Peroxodiphosphatverbindung (beispielsweise 25 bis 65 Gew.- %), mindestens ein Kupfersalz oder ein Kupferkomplex (beispielsweise 0,1 bis 8 Gew.- %) und mindestens eine Verbindung mit einem ionogen vorliegenden Halogenatom (beispielsweise 0,05 bis 7 Gew.- %). Exemplarisch seien als geeignete Bestandteile des vorangehend genannten Redoxsystems 1-Benzyl-5-Phenylbarbitursäure, Natriumperoxodisulphat, Kupferacetylacetonat und Benzyldibutylammoniumchlorid genannt.

Alternativ können auch Photoinitiatoren, welche mittels UV- oder mittels sichtbaren Lichts gestartet werden, zum Einsatz kommen. Geeignete Photoinitiatoren sind z.B. Dialkylbenzilketale, Dialkoxyacetophenone, Diacetyl, Campherchinon, Acetylphosphinoxide, Bisacylphosphinoxide, alpha-Diketone, Phoril, Anisil oder 4,4-Dichlorbenzil oder deren Mischungen.

Ferner kann man die Feststoffkomponente B und/oder die Flüssigkomponente A in bekannter Weise, je nach den Polymerisationsbedingungen (Kalt-/Heißpolymerisation), mit weiteren Zusatzstoffen versehen, beispielsweise mit mindestens einem Stabilisator, mindestens einem UV-Absorber, mindestens einem Thixotropiermittel, mindestens einem Füllstoff oder Mischungen der vorangehenden Komponenten.

Als Füllstoffe kommen z.B. in Betracht pyrogene oder Fällungskieselsäuren, Dentalgläser wie Aluminosilikatgläser oder Fluoroaluminosilikatgläser, Stronciumsilikat, Stronciumborsilikat, Schichtsilikate, Zeolithe, Metalloxide oder Fasern oder deren Mischungen. Geeignete Fasern umfassen z.B. Glasfasern sowie Polyamid- oder Kohlenstofffasern.

Des Weiteren können die Feststoffkomponente B und/oder die Flüssigkomponente A mit weiteren Additiven, beispielsweise anti-mikrobiellen Additiven, ausgestattet sein. Solche geeigneten biozid wirkenden Additive können z.B., insbesondere in Form einer Mischung, mindestens ein anorganisches Kupfersalz, mindestens ein anorganisches Silbersalz und mindestens ein anorganisches Bariumsalz sowie optional Silber umfassen. Dabei ist bevorzugt, dass das Kupfersalz Kupfersulphat, das Silbersalz Silberphosphat und das Bariumsalz Bariumsulphat darstellen. Gemäß einer weiteren, bevorzugten Ausgestaltung ist vorgesehen, dass das anorganische Kupfersalz, insbesondere Kupfersulphat, und/oder das anorganische Silbersalz, insbesondere Silberphosphat, und/oder das anorganische Bariumsalz, insbesondere Bariumsulphat, und/oder gegebenenfalls Silber, auf einem inerten Trägermaterial vorliegt bzw. vorliegen. Vorzugsweise liegen das anorganische Kupfersalz, insbesondere Kupfersulphat, das anorganische Silbersalz, insbesondere Silberphosphat, und das anorganische Bariumsalz, insbesondere Bariumsulphat, sowie gegebenenfalls Silber gemeinsam auf einem Träger vor. Geeignete inerte Trägermaterialen umfassen z.B. in partikulärer Form vorliegende Polymermaterialien und auch anorganische Substanzen, z.B. Silikate.

Die aus den beschriebenen erfindungsgemäßen Mehrkomponenten-Prothesenausgangsmaterialien erhältlichen Prothesenkörper bzw. -materialien zeichnen sich regelmäßig durch einen Restmonomergehalt kleiner 2,0 Gew.- %, bestimmt gemäß ISO 1567, aus.

Interessanter Weise lassen sich mit den erfindungsgemäßen Mehrkomponenten-Prothesenausgangsmaterialien zufriedenstellende Resultate hinsichtlich Bruchfestigkeit und Restmonomergehalt unabhängig davon erzielen, ob heiß- oder kaltpolymerisiert wird. Auch spielt die spezifische Verfahrensvariante, d.h. ob das Prothesenteil im Gießverfahren oder im Injektionsverfahren hergestellt wird, regelmäßig keine Rolle.

Zur besseren Veranschaulichung wird die vorliegende Erfindung anhand der nachfolgenden Beispiele im Detail erläutert. Selbstverständlich ist die vorliegende Erfindung nicht auf diese Beispiele beschränkt.

### Beispiele:

### 1. Im Gießverfahren aus einem Kalt(auto)polymerisat erhaltener Prothesenkörper

### a) erfindungsgemäß

Die Flüssigkomponente A setzte sich zusammen aus 94,8 Gew.-% Methylmethacrylat, 5,0 Gew.-% 1,4-Butandioldimethacrylat als Vernetzer und etwa 0,2 Gew.-% Trioctylmethylammoniumchlorid und Kupfernaphthenat als Komponenten des Initiatorsystems. Die Pulver- bzw. Feststoffkomponente B setzte sich zusammen aus einem Methylmethacrylatperlpolymerisat mit einer mittleren Teilchengröße von etwa 50 µm in einer Menge von etwa 88,5 Gew. %, einem vernetzten Methylmethacrylatperlpolymerisat aus Methylmethacrylat und Ethylenglykoldimethacrylat als Vernetzer mit einer mittleren Teilchengröße von etwa 8 µm in einer Menge von 10 Gew.-% und einem Barbitursäurederivat als weiterer Komponente des Initiatorsystems (etwa 1,5 Gew. %). Das Mischungsverhältnis (w/w) von Komponente B zu Komponente A betrug 10:5.

Die erhaltene Mischung war für etwa 3 min. gießfähig. Der Volumenschrumpf betrug etwa 7 %. Der Restmonomergehalt gemäß ISO 1567 wurde mit 1,9 bestimmt. Die Bruchfestigkeit gemäß ISO 1567 lag bei 75 und der E-Modul gemäß ISO 1567 lag bei 2550.

### b) nicht erfindungsgemäß

Die Flüssigkomponente A setzte sich zusammen aus 94,8 Gew.-% Methylmethacrylat, 5,0 Gew.-% 1,4-Butandioldimethacrylat als Vernetzer und etwa 0,2 Gew.-% Trioctylmethylammoniumchlorid und Kupfernaphthenat als Komponenten des Initiatorsystems.

Die Pulver- bzw. Feststoffkomponente B setzte sich zusammen aus einem Polymethylmethacrylatgemisch, enthaltend 63,5 Gew.-% an Perlpolymerisat mit einer mittleren Teilchengröße von etwa 40 µm, 25 Gew.-% an einem Perlpoymerisat mit einer mittleren Teilchengröße von etwa 64 µm und 10 Gew.-% an einem Perlpolymerisat mit einer mittleren Teilchengröße von etwa 66 µm und einem Barbitursäurederivat als weiterer Komponente des Initiatorsystems (etwa 1,5 Gew. %). Das Mischungsverhältnis (w/w) von Komponente B zu Komponente A betrug 10:7.

Die erhaltene Mischung war für etwa 3 min. gießfähig. Der theoretische Volumenschrumpf betrug etwa 9%. Der Restmonomergehalt gemäß ISO 1567 wurde mit 2,7 bestimmt. Die Bruchfestigkeit gemäß ISO 1567 lag bei 73 und der E-Modul gemäß ISO 1567 lag bei 2400.

### 2. Im Injektionsverfahren aus einem Kalt(auto)polymerisat erhaltener Prothesenkörper

### a) erfindungsgemäß

Die Flüssigkomponente A setzte sich zusammen aus 94,8 Gew.-% Methylmethacrylat, 5,0 Gew.-% 1,4-Butandioldimethacrylat als Vernetzer und etwa 0,2 Gew.-% Trioctylmethylammoniumchlorid und Kupfernaphthenat als Komponenten des Initiatorsystems. Die Pulver- bzw. Feststoffkomponente B setzte sich zusammen aus einem Methylmethacrylatperlpolymerisat mit einer mittleren Teilchengröße von etwa 50 µm in einer Menge von etwa 88,5 Gew. %, einem vernetzten Methylmethacrylatperlpolymerisat aus Methylmetharcrylat und Ethylenglykoldimethacrylat als Vernetzer mit einer mittleren Teilchengröße von etwa 8 µm in einer Menge von 10 Gew.-% und einem Barbitursäurederivat als weiterer Komponente des Initiatorsystems (etwa 1,5 Gew. %). Das Mischungsverhältnis (w/w) von Komponente B zu Komponente A betrug 10:4.

Die erhaltene Mischung war für etwa 3 min. in einen Injektionszylinder einfüllbar. Der theoretische Volumenschrumpf betrug etwa 6 %. Der Restmonomergehalt gemäß ISO 1567 wurde mit 1,7 bestimmt. Die Bruchfestigkeit gemäß ISO 1567 lag bei 80 und der E-Modul gemäß ISO 1567 lag bei 2400.

### b) nicht erfindungsgemäß

Die Flüssigkomponente A setzte sich zusammen aus 94,8 Gew.-% Methylmethacrylat, 5,0 Gew.-% 1,4-Butandioldimethacrylat als Vernetzer und etwa 0,2 Gew.-% Trioctylmethylammoniumchlorid und Kupfernaphthenat als Komponenten des Initiatorsystems.

Die Pulver- bzw. Feststoffkomponente B setzte sich zusammen aus einem Polymethylmethacrylatgemisch, enthaltend 63,5 Gew.-% an Perlpolymerisat mit einer mittleren Teilchengröße von etwa 40 µm, 25 Gew.-% an einem Perlpolymerisat mit einer mittleren Teilchengröße von etwa 64 µm und 10 Gew.-% an einem Perlpolymerisat mit einer mittleren Teilchengröße von etwa 66 µm und einem Barbitursäurederivat als weiterer Komponente des Initiatorsystems (etwa 1,5 Gew. %). Das Mischungsverhältnis (w/w) von Komponente B zu Komponente A betrug 10:5.

Die erhaltene Mischung war für etwa 3 min. in einen Injektionszylinder einfüllbar. Der theoretische Volumenschrumpf betrug etwa 7%. Der Restmonomergehalt gemäß ISO 1567 wurde mit 2,3 bestimmt. Die Bruchfestigkeit gemäß ISO 1567 lag bei 78 und der E-Modul gemäß ISO 1567 lag bei 2350.

### 3. Im Press-/Stopfverfahren aus einem Heißpolymerisat erhaltener Prothesenkörper

### a) erfindungsgemäß

Die Flüssigkomponente A setzte sich zusammen aus 95,0 Gew.-% Methylmethacrylat und 5,0 Gew.-% 1,4-Butandioldimethacrylat als Vernetzer. Die Pulver- bzw. Feststoffkomponente B setzte sich zusammen aus einem Methylmethacrylatperlpolymerisat mit einer mittleren Teilchengröße von etwa 86 µm in einer Menge von etwa 90 Gew. % und einem vernetzten Methylmethacrylatperlpolymerisat aus Methylmethacrylat und Ethylenglykoldimethacrylat als Vernetzer mit einer mittleren Teilchengröße von etwa 8 µm in einer Menge von 10 Gew.-%, wobei in dem Methylmethacrylatperlpolymerisat Dibenzoylperoxid als Initiator vorliegt (etwa 1 Gew. %), d.h. das Perlpolymerisat und der Initiator addieren sich auf 90 Gew.-%. Das Mischungsverhältnis (w/w) von Komponente B zu Komponente A betrug 10:3.

Die Anquellzeit betrug 4 bis 6 min, die Teigphase lag bei etwa 20 min. Der theoretische Volumenschrumpf betrug etwa 5 %. Der Restmonomergehalt gemäß ISO 1567 wurde mit 0,5 bestimmt. Die Bruchfestigkeit gemäß ISO 1567 lag bei 75 und der E-Modul gemäß ISO 1567 lag bei 2600.

### b) nicht erfindungsgemäß

Die Flüssigkomponente A setzte sich zusammen aus 95,0 Gew.-% Methylmethacrylat und 5,0 Gew.-% 1,4-Butandioldimethacrylat als Vernetzer.

Die Pulver- bzw. Feststoffkomponente B setzte sich zusammen aus einem Polymethylmethacrylatgemisch, enthaltend 70 Gew.-% an einem Polymethylmethacrylat-Perlpolymerisat mit einer mittleren Teilchengröße von etwa 86 µm und 30 Gew.-% an einem Polymethylmethacrylat-Perlpolymerisat mit einer mittleren Teilchengröße von etwa 40 µm, wobei in den Perlpolymerisaten insgesamt 1 Gew.-% an Dibenzolperoxid als Initiator vorliegt. Die Perlpolymerisate und der Initiator addieren sich auf 100 Gew.-%. Das Mischungsverhältnis (w/w) von Komponente B zu Komponente A betrug 10:4.

Die Anquellzeit betrug 5 bis 10 min, die Teigphase lag bei etwa 30 min. Der theoretische Volumenschrumpf betrug etwa 6 %. Der Restmonomergehalt gemäß ISO 1567 wurde mit 0,7 bestimmt. Die Bruchfestigkeit gemäß ISO 1567 lag bei 78 und der E-Modul gemäß ISO 1567 lag bei 2600.

Die in der vorstehenden Beschreibung und in den Ansprüchen offenbarten Merkmale der Erfindung können sowohl einzeln aus auch in jeder beliebigen Kombination für die Verwirklichung der Erfindung in ihren verschiedenen Ausfühmngsformen wesentlich sein.

## Patentansprüche

1. Polymerisierbares Mehrkomponenten-Prothesenausgangsmaterial, insbesondere 2-Komponenten-Prothesenausgangsmaterial, umfassend
a) mindestens eine flüssige Komponente A, enthaltend mindestens ein Alkylmethacrylat, insbesondere Methylmethacrylat,
b) mindestens eine Feststoffkomponente B, enthaltend
i) mindestens ein erstes Homo- und/oder Copolymer eines Alkyl(meth)acrylats, insbesondere des Methylmethacrylats, in Pulverform und/oder in Form von Perlen mit einer ersten durchschnittlichen Partikelgröße (auch erstes (Co)polymerisat genannt) und
ii) mindestens ein zweites Homo- und/oder Copolymer eines Alkyl(meth)acrylats, insbesondere des Methylmethacrylats, in Perlenform mit einer zweiten durchschnittlichen Partikelgröße (auch zweites (Co)polymerisat genannt), wobei die Perlen dieses zweiten (Co)polymerisats zumindest teilweise vernetzt vorliegen,
wobei die erste durchschnittliche Partikelgröße größer als die zweite durchschnittliche Partikelgröße ist, wobei die erste durchschnittliche Partikelgröße zwischen 30 µm und 125 µm liegt und wobei die zweite durchschnittliche Partikelgröße im Bereich von 0,1 µm bis 15 µm liegt.

2. Prothesenausgangsmaterial nach Anspruch 1, **dadurch gekennzeichnet, dass** die Perlen des ersten (Co)polymerisats zumindest teilweise vernetzt vorliegen.

3. Prothesenausgangsmaterial nach einem der vorangehenden Ansprüche, **dadurch gekennzeichnet, dass** die erste durchschnittliche Partikelgröße der Perlen des ersten (Co)polymerisats im Bereich von 35 µm und 80 µm liegt.

4. Prothesenausgangsmaterial nach einem der vorangehenden Ansprüche, **dadurch gekennzeichnet, dass** in der Feststoffkomponente B nicht mehr als 20 Gew.-%, an Komponente ii) und mindestens 80 Gew.-%, an Komponente i) vorliegen.

5. Prothesenausgangsmaterial nach einem der vorangehenden Ansprüche, **dadurch gekennzeichnet, dass** das Gewichtsverhältnis der Komponente B zu der Komponente A im Bereich von 10 : 8 bis 10 : 2, insbesondere im Bereich von 10 : 5,5 bis 10 : 2,5, liegt.

6. Prothesenausgangsmaterial nach einem der vorangehenden Ansprüche, **dadurch gekennzeichnet, dass** in der Komponente i) und/oder ii) mindestens ein Vernetzer, insbesondere im Bereich von 0,1 Gew.-% bis 10 Gew.-% oder im Bereich von 0,5 Gew.-% bis 5 Gew.-%, als vernetztes Comonomer einpolymerisiert vorliegt.

7. Prothesenausgangsmaterial nach einem der vorangehenden Ansprüche, **dadurch gekennzeichnet, dass** die Vernetzer Di-, Tri- und/oder Tetra(meth)acrylate darstellen.

8. Prothesenausgangsmaterial nach einem der vorangehenden Ansprüche, **dadurch gekennzeichnet, dass** der Vernetzer 1,4-Butandioldi(meth)acrylat, Ethylenglycoldi(meth)acrylat, Urethandi(meth)acrylat, acryliertes oder methacryliertes Butadien-Oligomer oder eine Mischung dieser Komponenten darstellt.

9. Prothesenausgangsmaterial nach einem der vorangehenden Ansprüche, **dadurch gekennzeichnet, dass** die Komponenten i) und/oder ii) ein mit mindestens einer elastischen Phase modifiziertes Perlpolymerisat darstellen.

10. Prothesenausgangsmaterial nach Anspruch 9, **dadurch gekennzeichnet, dass** die elastische Phase ausgewählt ist aus der Gruppe bestehend aus Poly-(n-butylacrylat), Butadien-Styrol-Copolymeren, Silikonkautschuk-Pfropfcopolymerisaten, Urethan(meth)acrylaten, acryliertem oder methacryliertem Butadien-Oligomer oder -Polymer, und acryliertem oder methacryliertem Acrylnitril-Butadien-Oligomer oder -Polymer oder Mischungen dieser Komponenten.

11. Prothesenausgangsmaterial nach einem der vorangehenden Ansprüche, **dadurch gekennzeichnet, dass** die Flüssigkomponente A) und/ oder die Feststoffkomponente B) mindestens ein antimikrobielles Additiv enthalten, insbesondere umfassend mindestens ein anorganisches Kupfersalz, mindestens ein anorganisches Silbersalz, mindestens ein anorganisches Bariumsalz und/oder Silber.

12. Prothesenausgangsmaterial nach einem der vorangehenden Ansprüche, **dadurch gekennzeichnet, dass** die Flüssigkomponente A mindestens eine zur Vernetzung befähigte Vernetzer-Comonomerkomponente und/oder mindestens einen Polymerisationsbeschleuniger enthält und/oder dass die Feststoffkomponente B mindestens einen Initiator enthält.

13. Prothesenausgangsmaterial nach Anspruch 12, **dadurch gekennzeichnet, dass** der Initiator mindestens ein Peroxid, Hydroperoxid, Perketal, Perester oder eine Azoverbindung oder eine Mischung der genannten Komponenten oder ein Redoxsystem, enthaltend neben mindestens einem Peroxid, Perketal, Perester und/oder einer Azoverbindung auch noch Ascorbinsäure oder deren Derivate, Barbitursäure oder ein Barbitursäurederivat, Thiobarbitursäure oder ein Thiobarbitursäurederivat, eine Sulfinsäure oder deren Mischungen, umfasst.

14. Prothesenausgangsmaterial nach einem der vorangehenden Ansprüche, **dadurch gekennzeichnet, dass** die flüssige Komponente A und/oder die Feststoffkomponente B ferner mindestens einen Stabilisator, mindestens einen UV-Absorber, mindestens ein Thixotropiermittel, mindestens einen Füllstoff oder Mischungen der vorangehend genannten Komponenten enthalten.

15. Prothesenausgangsmaterial nach einem der vorangehenden Ansprüche, **dadurch gekennzeichnet, dass** das zweite (Co)polymerisat gebildet ist aus einer Comonomermischung, umfassend Methylmethacrylat, n-Butylacrylat und mindestens ein Vernetzercomonomer, insbesondere Ethylenglykoldimethacrylat.

16. Prothesenausgangsmaterial nach einem der vorangehenden Ansprüche, **dadurch gekennzeichnet, dass** in der Feststoffkomponente B) i) und/oder der Feststoffkomponente B ii) ein Gehalt an reaktiver Initiatorkomponente, insbesondere in Form von Peroxiden, Perketalen, Perestern und/oder Azoverbindungen, vorliegt.

17. Prothesenmaterial, insbesondere Dentalprothesen, hergestellt aus mindestens einem Mehrkomponenten-Prothesenausgangsmaterial gemäß einem der vorangehenden Ansprüche.

18. Prothesenmaterial nach Anspruch 17, **dadurch gekennzeichnet, dass** dieses ein Prothesenbasismaterial, insbesondere für Dentalprothesen, darstellt.

19. Verwendung eines Prothesenausgangsmaterials gemäß einem der Ansprüche 1 bis 16 zur Herstellung von Prothesenmaterial, insbesondere von Dentalprothesen.

## Claims

1. A polymerizable multi-component prosthesis starting material, particularly a two-component prosthesis starting material, comprising
a) at least one liquid component A containing at least one alkyl methacrylate, particularly methyl methacrylate,
b) at least one solid component B containing
i) at least one first homo and/or copolymer of an alkyl (meth)acrylate, particularly of methyl methacrylate, in the form of a powder and/or in the form of pearls having a first average particle size (also called first (co)polymer), and
ii) at least one second homo- and/or copolymer of an alkyl (meth)acrylate, particularly of methyl methacrylate, in the form of pearls having a second average particle size (also called second (co)polymer), wherein the pearls of said second (co)polymer are present at least partially cross-linked,
wherein the first average particle size is greater than the second average particle size, wherein the first average particle size ranges between 30 µm and 125 µm and wherein the second average particle size ranges from 0,1 µm to 15 µm.

2. The prosthesis starting material according to claim 1, **characterized in that** the pearls of the first (co)polymer are present at least partially cross-linked.

3. The prosthesis starting material according to any one of the preceding claims, **characterized in that** the first average particle size of the pearls of the first (co)polymer ranges from 35 µm to 80 µm.

4. The prosthesis starting material according to any one of the preceding claims, **characterized in that** not more than 20 wt% of component ii) and at least 80 wt% of component i) are present in the solid component B.

5. The prosthesis starting material according to any one of the preceding claims, **characterized in that** the weight ratio of component B to component A ranges from 10 : 8 to 10: 2, particularly from 10 : 5.5 to 10 : 2.5.

6. The prosthesis starting material according to any one of the preceding claims, **characterized in that** at least one cross-linking agent, particularly ranging from 0,1 wt% to 10 wt% or from 0,5 wt% to 5 wt%, is present in component i) and/or ii) polymerized as a cross-linked comonomer.

7. The prosthesis starting material according to any one of the preceding claims, **characterized in that** the cross-linking agents are di-, tri-, and/or tetra(meth)acrylates.

8. The prosthesis starting material according to any one of the preceding claims, **characterized in that** the cross-linking agent is 1,4-butanediol di(meth)acrylate, ethylene glycol di(meth)acrylate, urethane di(meth)acrylate, acrylated or methacrylated butadiene oligomer or a mixture of said components.

9. The prosthesis starting material according to any one of the preceding claims, **characterized in that** components i) and/or ii) is a pearl polymer modified with at least one elastic phase.

10. The prosthesis starting material according to claim 9, **characterized in that** the elastic phase is selected from the group comprised of poly-(n-butyl acrylate), butadiene-styrene copolymers, silicone rubber graft copolymers, urethane (meth)acrylates, acrylated or methacrylated butadiene oligomer or polymer and acrylated or methacrylated acrylonitrile-butadiene oligomer or polymer or mixtures of said components.

11. The prosthesis starting material according to any one of the preceding claims, **characterized in that** the liquid component A) and/or the solid component B) contain at least one antimicrobial additive, particularly comprising at least one organic copper salt, at least one inorganic silver salt, at least one inorganic barium salt and/or silver.

12. The prosthesis starting material according to any one of the preceding claims, **characterized in that** the liquid component A) contains at least one cross-linking comonomer component capable of cross-linking and/or at least one polymerization accelerator and/or that the solid component B contains at least one initiator.

13. The prosthesis starting material according to claim 12, **characterized in that** the initiator contains at least one peroxide, hydroperoxide, perketal, perester or azo compound or a mixture of the named components or a redox system, comprising in addition to at least one peroxide, perketal, perester and/or azo compound, also ascorbic acid or derivatives thereof, barbituric acid or a barbituric acid derivative, thiobarbituric acid or a thiobarbituric acid derivative, sulphinic acid or mixtures thereof.

14. The prosthesis starting material according to any one of the preceding claims, **characterized in that** the liquid component A and/or the solid component B furthermore contain at least one stabilizer, at least one UV absorber, at least one tixotropic agent, at least one filler or mixtures of the aforementioned components.

15. The prosthesis starting material according to any one of the preceding claims, **characterized in that** the second (co)polymer is formed from a comonomer mixture, comprising methyl methacrylate, n-butyl acrylate and at least one cross-linking comonomer, particularly ethylene-glycol dimethacrylate.

16. The prosthesis starting material according to any one of the preceding claims, **characterized in that** a content of reactive initiator component, particularly in the form of peroxides, perketals, peresters and/or azo compounds, is present in the solid component B) i) and/or solid component B ii).

17. A prosthesis material, particularly dental prostheses, produced from at least one multicomponent prosthesis starting material according to any one of the preceding claims.

18. The prosthesis material according to claim 17, **characterized in that** the same is a prosthesis base material, particularly for dental prostheses.

19. Use of a prosthesis starting material according to any one of claims 1 to 16 for producing prosthesis material, particularly of dental prostheses.

## Revendications

1. Matériau de départ multicomposant polymérisable pour prothèses, notamment matériau de départ bi-composant pour prothèses, comprenant :
a) au moins un composant liquide A contenant au moins un méthacrylate d'alkyle, notamment un méthacrylate de méthyle,
b) au moins un composant solide B contenant
i) au moins un premier homo et/ou copolymère d'un (méth)acrylate d'alkyle, notamment du méthacrylate de méthyle, sous forme de poudre et/ou sous forme de perles avec une première taille moyenne de particule (également nommé premier (co)polymère) et
ii) au moins un second homo et/ou copolymère d'un (méth)acrylate d'alkyle, notamment du méthacrylate de méthyle, sous forme de perles avec une seconde taille moyenne de particule (aussi nommé second (co)polymère), dans lequel les perles du dit second (co)polymère sont présentes au moins partiellement réticulées,
dans lequel la première taille moyenne de particule est plus grande que la seconde taille moyenne de particule, la première taille moyenne de particule se situant entre 30 µm et 125 µm et la seconde taille moyenne de particule se situant dans une plage de 0,1 µm à 15 µm.

2. Matériau de départ pour prothèses selon la revendication 1, **caractérisé en ce que** les perles du premier (co)polymère sont présentes au moins partiellement réticulées.

3. Matériau de départ pour prothèses selon l'une quelconque des revendications précédentes, **caractérisé en ce que** la première taille moyenne de particule des perles du premier (co)polymère se situe dans une plage de 35 µm à 80 µm.

4. Matériau de départ pour prothèses selon l'une quelconque des revendications précédentes, **caractérisé en ce que**, dans le composant solide B, la présence du composant ii) ne dépasse pas 20 %m et celle du composant i) soit au moins de 80 %m.

5. Matériau de départ pour prothèses selon l'une quelconque des revendications précédentes, **caractérisé en ce que** le rapport massique du composant B au composant A se situe dans une plage de 10 : 8 à 10 : 2, notamment dans la tranche de 10 : 5,5 à 10 : 2,5.

6. Matériau de départ pour prothèses selon l'une quelconque des revendications précédentes, **caractérisé en ce que**, dans le composant i) et/ou ii), est présent au moins un agent réticulant, notamment dans la tranche de 0,1 %m à 10 %m ou dans la tranche de 0,5 %m à 5 %m, polymérisé en tant que comonomère réticulé.

7. Matériau de départ pour prothèses selon l'une quelconque des revendications précédentes, **caractérisé en ce que** les agents réticulants constituent des di, tri et/ou tetra(méth)acrylates.

8. Matériau de départ pour prothèses selon l'une quelconque des revendications précédentes, **caractérisé en ce que** l'agent réticulant constitue du butane-1,4-diol di(méth)acrylate, du di(méth)acrylate d'éthylène glycol, di(méth)acrylate d'uréthane, oligomère de butadiène acrylé ou méthacrylé ou un mélange des dits composants.

9. Matériau de départ pour prothèses selon l'une quelconque des revendications précédentes, **caractérisé en ce que** les composants i) et/ou ii) constituent un polymère en perles modifié avec au moins une phase élastique.

10. Matériau de départ pour prothèses selon la revendication 9, **caractérisé en ce que** la phase élastique est choisie parmi le groupe composé de poly-(n-butylacrylate), copolymères de styrène-butadiène, copolymère ramifiés caoutchouc silicone, (méth)acrylates d'uréthane, oligomère ou polymère de butadiène acrylé ou méthacrylé et oligomère ou polymère d'acrylonitrile butadiène acrylé ou méthacrylé ou des mélanges des dits composants.

11. Matériau de départ pour prothèses selon l'une quelconque des revendications précédentes, **caractérisé en ce que** le composant liquide A) et/ou le composant solide B) contiennent au moins un additif antimicrobien, notamment comprenant au moins un sel de cuivre organique, au moins un sel d'argent inorganique, au moins un sel de baryum inorganique et/ou de l'argent.

12. Matériau de départ pour prothèses selon l'une quelconque des revendications précédentes, **caractérisé en ce que** le composant liquide A) contient au moins un composant comonomère réticulant apte à la réticulation et/ou au moins un agent accélérateur de polymérisation et/ou **en ce que** le composant solide B contient au moins un initiateur.

13. Matériau de départ pour prothèses selon la revendication 12, **caractérisé en ce que** l'initiateur contient au moins un peroxyde, peroxyde d'hydrogène, perketal, perester ou un composé azo ou un mélange des composant nommés ou encore un système redox, comprenant, outre au moins un peroxyde, perketal, perester ou un composé azo, de plus un acide ascorbique ou ses dérivées, un acide barbiturique ou un dérivé d'acide barbiturique, un acide thiobarbiturique ou un dérivé d'acide thiobarbiturique, un acide sulfinique ou ses mélanges.

14. Matériau de départ pour prothèses selon l'une quelconque des revendications précédentes, **caractérisé en ce que** le composant liquide A et/ou le composant solide B contiennent en outre au moins un stabilisateur, au moins un absorbant UV, au moins un agent de thixotropie, au moins une matière de charge ou des mélanges des composants pré-cités.

15. Matériau de départ pour prothèses selon l'une quelconque des revendications précédentes, **caractérisé en ce que** le second (co)polimère est formé à partir d'un mélange de comonomères, comprenant du méthacrylate de méthyle, acrylate de n-butyle et au moins un comonomère réticulant, notamment diméthacrylate d'éthylène-glycol.

16. Matériau de départ pour prothèses selon l'une quelconque des revendications précédentes, **caractérisé en ce que**, dans le composant solide B) i) et/ou le composant solide B ii), est présent un contenu de composant initiateur réactif, notamment sous forme de peroxydes, perketals, peresters et/ou composés azo.

17. Matériau pour prothèses, notamment prothèses dentaires, fabriqué à partir d'au moins un matériau de départ multicomposant pour prothèses selon l'une quelconque des revendications précédentes.

18. Matériau pour prothèses selon la revendication 17, **caractérisé en ce que** celui-ci constitue un matériau de base pour prothèses, notamment prothèses dentaires.

19. Utilisation d'un matériau de départ pour prothèses selon l'une des revendications 1 à 16 pour la fabrication de matériau pour prothèses, notamment prothèses dentaires.
